(19) **Europäisches Patentamt**
**European Patent Office**
**Office européen des brevets**

(11) **EP 3 461 803 A1**

(12) **EUROPÄISCHE PATENTANMELDUNG**

(43) Veröffentlichungstag:
**03.04.2019 Patentblatt 2019/14**

(51) Int Cl.:
***C05C 3/00*** *(2006.01)* ***C05F 11/10*** *(2006.01)*

(21) Anmeldenummer: **17194295.6**

(22) Anmeldetag: **02.10.2017**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA MD**

(71) Anmelder: **Evonik Degussa GmbH**
**45128 Essen (DE)**

(72) Erfinder:
• **Jakob, Harald**
**63594 Hasselroth (DE)**
• **Hasselbach, Hans Joachim**
**63571 Gelnhausen (DE)**
• **Egly, Dominik**
**64720 Michelstadt (DE)**
• **Hessberger, Waldemar**
**63755 Alzenau (DE)**

(54) **HERSTELLVERFAHREN FÜR DIPEPTIDHALTIGE GRANULATE**

(57)     Die vorliegende Erfindung betrifft ein Verfahren zur Herstellung einer Methionin, Methionylmethionin, Kalium in Form von Kaliumsalz und Ammoniumsulfat enthaltenden, partikelförmigen Zusammensetzung sowie deren Verwendung.

**EP 3 461 803 A1**

**Beschreibung**

Die vorliegende Erfindung betrifft insbesondere ein Verfahren zur Herstellung einer Methionin, Methionylmethionin, Kaliumsalz und Ammoniumsulfat enthaltende, partikelförmigen Zusammensetzung sowie deren Verwendung.

Die Aminosäure Methionin wird gegenwärtig weltweit in großen Mengen industriell hergestellt und ist von beträchtlicher kommerzieller Wichtigkeit. Methionin wird auf vielen Gebieten angewendet, wie zum Beispiel für pharmazeutische, Gesundheits- und Fitnessprodukte, insbesondere jedoch als Futtermitteladditiv in vielen Futtermitteln für verschiedene Nutztiere. Im industriellen Maßstab wird Methionin chemisch über die Bucherer-Bergs-Reaktion hergestellt, die eine Variante der Strecker-Synthese darstellt. Dabei werden die Ausgangssubstanzen 3-Methylmercaptopropanal (hergestellt aus 2-Propenal und Methylmercaptan), Blausäure (Cyanwasserstoff), Ammoniak und Kohlendioxid zum 5-(2-Methylmercaptoethyl)-hydantoin (Methioninhydantoin) umgesetzt und dieses anschließend alkalisch mit Kaliumcarbonat und Kaliumhydrogencarbonat zum Kaliummethioninat hydrolysiert. Methionin wird schließlich durch Behandlung mit Kohlendioxid aus seinem Kaliumsalz freigesetzt, welches als Präzipitat aus der Kaliumcarbonat und Kaliumhydrogencarbonat enthaltenden Mutterlauge abfiltriert werden kann (US 5,770,769). Die Reagenzien Ammoniak, Kaliumcarbonat und Kaliumhydrogencarbonat sowie Kohlendioxid werden bei der industriellen Methioninproduktion in der Regel rezykliert. Von Zeit zu Zeit ist es aber erforderlich, die wässrige Mutterlauge dieses Hydantoinhydrolysekreislaufs teilweise gegen frisches Kaliumhydroxid auszutauschen, im Wesentlichen etwa um das in Form von neutralem Kaliumformiat inaktivierte Kaliumsalz aus dem Kreislauf zu entfernen ("Purge"). Kaliumformiat bildet sich aus den in der Methioninhydantoinlösung vorhandenen Resten an Blausäure und Kaliumsalzen aus der Hydantoinhydrolyse (WO2013030068A2). Ein weiteres Nebenprodukt der Methioninsynthese ist das Dipeptid Methionylmethionin (EP 1 564 208 A1). Generell muss die übermäßige Anreicherung von Nebenprodukten im Hydantoinhydrolysekreislauf vermieden werden, da ansonsten Störungen der Kristallbildung im Downstream auftreten.

Diese sogenannte Purge-Lösung enthält ungefähr 2 bis 6 Gew.% Methionin, 4 bis 8 Gew.% Methionylmethionin und 6 bis 14 Gew.% Kalium in Form von Kaliumsalzen. Wegen des Gehaltes an Kalium, Stickstoff und Schwefel eignet sich diese Lösung als Flüssigdünger (C.C. Mitchel und A.E. Hiltbold, Journal of Plant Nutrition, 17(12), 2119-2134, 1994). Wünschenswert war jedoch, einen derartigen Dünger in fester Form bereitzustellen. Versuche, diese Lösung zu einem festen, rieselfähigen Feststoffgemisch zu entwässern, um diesen Wertstoff lager- und transportfähiger zu machen, waren jedoch bisher fehlgeschlagen (vgl. Beispiel 6 in diesem Dokument).

Die der vorliegenden Erfindung grundsätzlich zugrundeliegende Aufgabe war demzufolge die Bereitstellung eines Feststoffdüngers auf Basis von Methionin und Kaliumsalzen sowie ein einfaches und kostengünstiges Verfahren zu seiner Herstellung, bei dem insbesondere auch die wässrige Mutterlauge des oben beschriebenen Hydantoinhydrolysekreislaufs als Wertstoff verwendbar ist.

Diese grundlegende Aufgabe wurde gelöst durch das Auffinden eines Verfahrens, bei dem ein Gas zusammen mit der wässrigen Ausgangsmischung, enthaltend Methionin, Methionylmethionin, Kalium in Form von Kaliumsalz und Ammoniumsulfat,

als Flüssigkomponente über eine Düse gesprüht wird. Die wässrige Ausgangsmischung wurde dabei zunächst erzeugt aus der o.g. Purge-Lösung durch gezielte Zugabe von Ammoniumsulfatlösung. Bei der anschließenden Sprühgranulation der wässrigen Ausgangsmischung zu einer rieselfähigen, lagerstabilen Feststoffzusammensetzung wurde bevorzugt eine Zweistoffdüse verwendet. Dieses Verfahren ist beschrieben in der bis dato unveröffentlichten EP-Anmeldung mit Anmeldenummer 16176371.9. Die Verwendung einer Zweistoffdüse für die Sprühgranulation erfordert aber eine vorherige Mischung der Ausgangslösungen, nämlich Purge-Lösung und wässriger Ammoniumsulfatlösung. Wenn man zusätzlich eine pH-Einstellung in den sauren Bereich durch die Zugabe von $H_2SO_4$ vornimmt, dann wird Wärme erzeugt und CO freigesetzt, was ein Schäumen der Mischung initiiert. Weiterhin fällt ein klebriger Feststoff aus, welcher größtenteils aus Met-Met und Methionindiketopiperazin besteht. Diese klebrige Phase führt dazu, dass sich die Düsen, Rohrleitungen und die Rührbehälter mit der Zeit zusetzen. Um ein Verkleben der Düse und der Rohrleitungen zu vermeiden, kann die klebrige Phase vor dem Versprühen mithilfe eines Hochleistungsdispergators in feine Tröpfchen zerteilt werden, was jedoch wieder zusätzlichen Aufwand bedeutet.

Die in der EP-Anmeldung mit Anmeldenummer 16176371.9 beschriebenen Versuche zeigen ferner, dass die feinen Tröpfchen der klebrigen Phase mithilfe der Sprühgranulationstechnik in das Endprodukt eingeschlossen werden. Die Entstehung der klebrigen Phase erfordert allerdings eine regelmäßige Reinigung der Vorlagebehälter, da sich über einen längeren Zeitraum Wandbeläge aufbauen können.

Die im Speziellen zu lösende Aufgabe war daher ein Sprühgranulationsverfahren zu finden, welches die vorstehend genannten Nachteile nicht oder nur in verringertem Ausmaß aufweist. Insbesondere sollte auf möglichst einfache und direkte Art und Weise aus der Purge-Lösung und der wässrigen Ammoniumsulfatlösung eine rieselfähige, lagerstabile Feststoffzusammensetzung der vorstehend bezeichneten Art hergestellt werden können.

Die genannte Aufgabe wird gelöst durch ein Verfahren der Sprühgranulation zur Herstellung einer Methionin, Methionylmethionin, Kaliumsalz und Ammoniumsulfat enthaltenden, partikelförmigen Zusammensetzung, dadurch gekennzeichnet dass

(a) eine erste wässrige Lösung oder Suspension (i), enthaltend

30 bis 40 Gew.% Ammoniumsulfat,
eine zweite wässrige Lösung oder Suspension (ii), enthaltend
2 bis 6 Gew.% , vorzugsweise 3 bis 5 Gew. % Methionin,
4 bis 8 Gew.%, vorzugsweise 5 bis 7 Gew. % Methionylmethionin und
6 bis 14 Gew.%, vorzugsweise 8 bis 12 Gew. % Kalium in Form von Kaliumsalz und
ein Gas über eine Dreistoffdüse in eine Wirbelschicht versprüht werden zur Erzeugung von Rohpartikeln unter gleichzeitigem Verdampfen von Wasser und optional

(b) die in Schritt (a) erhaltenen Rohpartikel getrocknet werden.

Die beiden wässrigen Lösungen oder Suspensionen (i) und (ii) können je nach verwendeter Konzentration mehr oder weniger ungelöste Bestandteile aufweisen.

Die zweite wässrige Lösung oder Suspension (ii) kann auch nennenswerte Mengen Natrium in Form von Natriumsalz enthalten, in der Regel bis zu 4 Gew.%, vorzugsweise bis zu 3 Gew% Natrium. Dies ist insbesondere dann der Fall, wenn zuvor Mischungen aus Kalium- und Natriumhydroxid, Kalium- und Natriumcarbonat und/oder Kalium- und Natriumhydrogencarbonat als Verseifungsagenz in der alkalischen Hydrolysereaktion von Methioninhydantoin zu Alkalimethioninat verwendet worden sind, wie es z.B. in der EP-Patentanmeldung mit EP-Anmeldenummer 17171060.1 beschrieben worden ist.

Das Verfahren ist in jedem Falle einfach und kostengünstig in gängigen Apparaturen durchzuführen.

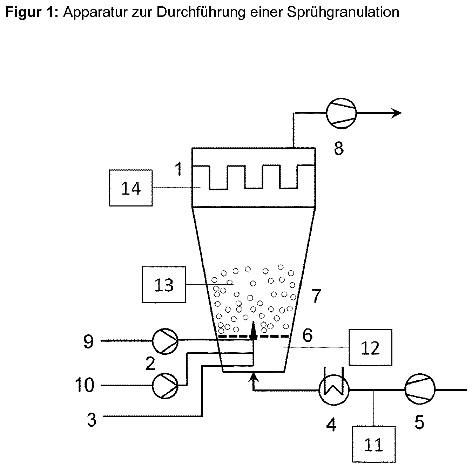

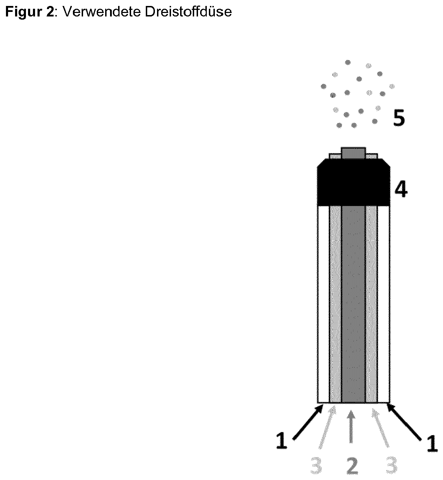

Mithilfe der Dreistoffdüse müssen die Ausgangsmedien, Purge-Lösung (zweite wässrige Lösung oder Suspension ii) bzw. Ammoniumsulfatlösung (erste wässrige Lösung oder Suspension i), vorteilhafterweise nicht vor dem Versprühen in einem Mischbehälter vorgemischt werden, sondern die Mischung findet kurz nach der Düse statt. In Figur 2 ist das Prinzip einer Dreistoffdüse dargestellt. Die Düse besitzt zwei Eingänge (Stutzen), für die flüssigen Ausgangsmedien und einen Eingang für das Gas (z.B. Düsenluft). Dabei werden die zu versprühenden Lösungen z.B. mit einer geeigneten Pumpe der Düsenvorrichtung zugeführt. Die beiden Kanäle für den Durchfluss der Lösungen zur Düse hin sind zum einen als Zentralrohr, ggf. mit Verlängerung, und zum anderen als innerer Ringspalt zwischen dem Zentralrohr und einem dieses Zentralrohr umhüllenden inneren Mantelrohr angelegt. Den Kanal für das Gas wiederum bildet ein äußerer Ringspalt zwischen dem inneren Mantelrohr und einem das innere Mantelrohr umhüllenden äußeren Mantelrohr, wie es z.B. in Fig.1 der EP 716640 B1 gezeigt ist. Die Auslässe aller drei Kanäle münden dabei in den Düsenkopf. Es können grundsätzlich auch andere Ausführungsformen von Dreistoffdüsen verwendet werden, die der hier gezeigten prinzipiellen Funktionsweise entsprechen. Auch verwendbar sind Vierstoffdüsen, welche als Dreistoffdüse betrieben werden. Bei Verwendung einer Vierstoffdüse kann auch so vorgegangen werden, dass durch den vierten Eingang die Schwefelsäure zum Ansäuern der Ammoniumsulfatlösung oder Suspension (i) zugeführt wird. Das hat den Vorteil, dass der Schritt des Ansäuerns der Ammoniumsulfatlösung im Vorfeld entfallen kann und dafür direkt in situ während der Sprühgranulation erfolgt.

Über den Stutzen zum Zentralrohr wird die erste wässrige Lösung oder Suspension (i) (Ammoniumsulfatlösung ggf. versetzt mit zusätzlicher $H_2SO_4$) aufgegeben und die Zugabe der zweiten wässrigen Lösung oder Suspension (ii) (z.B. Purge-Lösung) erfolgt über den zweiten Stutzen zum inneren Ringspalt. Die beiden Kanäle für die Zufuhr der beiden wässrigen Lösungen oder Suspensionen (i und ii) können auch untereinander ausgetauscht werden. Das Gas hingegen wird über den dritten Stutzen in den äußeren Ringspalt eingetragen, dem dafür geeigneten Kanal und hat die Aufgabe, die flüssigen Medien in feine Tröpfchen zu zerstäuben, um diese in den Prozessraum zu verteilen und z.B. in das dort vorhandene Wirbelbett (aufgewirbelter Feststoff) zu sprühen. Das Gas, vorzugsweise heiße Luft oder Stickstoff, wird dabei gleichzeitig von unten nach oben in den Apparat geströmt, um erstens das vorhandene Material zu fluidisieren und zweitens die versprühte Flüssigkeit zu verdampfen (Fig. 1).

Der versprühte Feststoff bleibt idealerweise auf den vorhandenen Partikeln haften, wodurch sich ein diskretes Partikelwachstum einstellen lässt. Die im Austrag zu erzielende Korngröße ist von der Keimbilanz in der Wirbelschichtanlage abhängig. Diese wird wesentlich vom Gleichgewicht von Keimbildung durch Abrieb oder nicht treffende Sprühtropfen und dem Granulataufbau bestimmt. Gezielt kann die Korngröße durch die Wahl der Trocknungs-, der Sprühparameter und durch den Einsatz eines Zerhackers in der Wirbelschicht eingestellt werden. Die so erzeugten Granulate können durch klassierende Einrichtungen (z.B. Sichter und Unterlaufwehr) in angestrebter Partikelgröße kontinuierlich aus dem Trocknungsraum ausgetragen werden.

Bevorzugt wird dabei eine Verfahrensweise, bei welcher die wässrige Lösung oder Suspension (ii) in einem Gewichtsverhältnis zur wässrigen Lösung oder Suspension (i) von 1,0/0,5 bis 1,0/3,0 eingesetzt wird, weil zum einen die gewünschte Partikelzusammensetzung damit gut erreichbar ist und zum anderen die Sprühgranulation vorteilhaft betrieben werden kann.

Desweiteren bevorzugt wird Luft oder Stickstoff als Gas verwendet, da beide günstig verfügbar sind und das dabei entstehende Abgas problemlos einer Verbrennung zugeführt werden kann.

Vorzugsweise wird das Sprühgranulationsverfahren a) in einer Wirbelschicht durchgeführt, insbesondere bei einer Temperatur in der Wirbelschicht von 60 bis 130°C, weil zum einen die gewünschten Partikeleigenschaften damit gut erreichbar sind und zum anderen die Sprühgranulation vorteilhaft betrieben werden kann.

Weitere Vorteile des unter Einsatz einer Dreistoffdüse durchgeführten Sprühgranulationsverfahrens sind:

- Kein Mischbehälter und Kühlung des Behälters für die vorherige Mischung und pH-Einstellung der Ausgangsmedien ist erforderlich.
- Schaumbildung und Entstehung der klebrigen Phase wird vermieden.
- Die Wärmeentwicklung durch die pH Einstellung mit $H_2SO_4$ kann für den Granulationsprozess genutzt werden.
- Verbesserung der Produkteigenschaften wie Hygroskopizität und Lagerstabilität wird erreicht
- Im Gegensatz zu den mit einer Zweistoffdüse erhaltenen Granulaten (beschrieben in EP 16176371.9) haben die erfindungsgemäß erzeugten Granulate eine deutlich reduzierte Verklumpungsneigung und geringere Hygroskopizität.

Als zweite wässrige Lösung oder Suspension (ii) kann dabei vorteilhafterweise direkt die aus dem eingangs erwähnten Verfahren zur Herstellung von Methionin durch Abtrennung erhältliche wässrige Mutterlauge verwendet werden, wobei das Verfahren mindestens die Schritte Hydrolyse von 5-(2-Methylmercaptoethyl)-hydantoin mit Kaliumcarbonat, Kaliumhydrogencarbonat zu wässrigem Kaliummethioninat (Hydantoinhydrolysekreislauf bei der industriellen Methioninproduktion),
Neutralisation von wässrigem Kaliummethioninat mit Kohlendioxid unter Bildung von Methionin und anschließende Kristallisation des Methionins umfasst.
Die wertstoffhaltige Mutterlauge wird dadurch in umweltfreundlicher Weise einer sinnvollen Verwertung zugeführt.

Die Ammoniumsulfat enthaltende, erste wässrige Lösung oder Suspension (i) kann vorteilhafterweise direkt aus der für die großtechnische Methioninproduktion notwendigen Herstellung von Blausäure aus Methan und Ammoniak, etwa nach dem Andrussow- Verfahren (US 8,802,020 B2) oder dem BMA-Verfahren (F. Endter, Chemie-Ing.-Techn. 30, 1958, Nr. 5, 305-310), stammen und entsteht im Wesentlichen durch Behandeln eines dabei jeweils anfallenden Blausäure und Ammoniak enthaltenden Gasgemisches mit wässriger Schwefelsäure (Schwefelsäure-Wäsche) und anschließender Neutralisation der erhaltenen wässrigen Lösung mit Ammoniak. Diese Lösung wird dadurch gleichzeitig in umweltfreundlicher Weise einer sinnvollen Verwertung zugeführt.
Auf diese Weise werden gleich zwei zusätzlich anfallende Wertstoffströme der industriellen Methioninproduktion ohne die Notwendigkeit von weiteren Additiven in ein neues vorteilhaftes Produkt überführt.

Beim Kaliumsalz kann es sich sowohl um ein Salz oder mehrere Salze von anorganischen als auch von organischen Säuren handeln, beispielsweise um mindestens ein Kaliumsalz ausgewählt aus der Gruppe umfassend Ameisensäure, Essigsäure, Propansäure, 2-Hydroxypropansäure, 2-Hydroxy-4-methylthiobutansäure aber auch Methionin und Methionylmethionin bei entsprechendem pH-Wert, Kaliumhydrogencarbonat, Kaliumcarbonat, Kaliumhydrogensulfat und Kaliumsulfat. Sofern verfahrensbedingt nennenswerte Mengen Natrium in Form von Natriumsalz enthalten sind, handelt es sich dabei insbesondere um die den hier genannten Kaliumsalzen entsprechenden Natriumsalze.

Die erste wässrige Lösung oder Suspension (i), z.B. Ammoniumsulfatlösung, wird bevorzugt vor ihrer weiteren Verarbeitung in Schritt (a) mit gerade so viel Schwefelsäure versetzt, dass nach der Sprühgranulation eine partikelförmigen Zusammensetzung erhalten wird, die nach Auflösen in Wasser einen pH-Wert von 3 bis 6, vorzugsweise von 3,5 bis 5,5 aufweist, gemessen bei Raumtemperatur an einer 10 Gew.%igen Lösung der partikelförmigen Zusammensetzung in Wasser mittels einer Glas-pH-Elektrode mit Flüssig-Elektrolyt (3-molare KCl Lösung)-Füllung.
Die genaue Menge der zuzusetzenden Schwefelsäure hängt insbesondere von dem bereits vorhandenen pH-Wert der verwendeten wässrigen Lösungen oder Suspensionen (i) und (ii) ab und kann jeweils leicht in Vorversuchen ermittelt werden.

Bei der Verarbeitung der Lösungen oder Suspensionen (i) und (ii) in Schritt (a) entsteht aus Ammoniumsulfat und Kaliumcarbonat teilweise Ammoniumcarbonat, welches beim Erhitzen zu Ammoniak und Kohlendioxid zerfällt und in Form dieser Gase entweicht. Durch Zugabe von Schwefelsäure zur Lösung oder Suspension (i) wird die Freisetzung von Ammoniak größtenteils verhindert und dieser wieder in Form von Ammoniumsulfat gebunden, wobei im Wesentlichen lediglich Kohlendioxid entweicht. Die Einstellung des pH-Werts auf 3 bis 6 bewirkt ferner eine Verbesserung der Lagerstabilität der in Schritt (b) des erfindungsgemäßen Verfahrens erhaltenen Partikel. Das Abscheiden einer klebrigen Phase wird dadurch in vorteilhafter Weise zusätzlich verhindert.

Bei einer vorteilhaften Ausgestaltung des erfindungsgemäßen Verfahrens wird die in Schritt (a) eingesetzte erste wässrige Lösung oder Suspension (i) vor Schritt (a) durch Verdampfen des Wassers zu einer wässrigen Suspension mit bis zu 70 Gew.% Feststoffgehalt aufkonzentriert. Dieses Konzentrat bildet in der Regel eine stabile Suspension. Durch das Aufkonzentrieren werden Energiekosten im Verfahren gespart.

Das erfindungsgemäße Verfahren kann sowohl im diskontinuierlichen als auch im kontinuierlichen Verfahren durchgeführt werden, beispielsweise in einem Wirbelschicht- bzw. Fließbettgranulator oder Mischgranulator.

Die Sprühgranulation wird dabei vorzugsweise in einer Wirbelschicht durchgeführt, wahlweise nach dem Bottom-Spray- oder Top-Sprayverfahren. Ein solches Verfahren ermöglicht die Bildung besonders gleichförmiger Teilchen der erfindungsgemäßen, partikelförmigen Zusammensetzung mit relativ enger Korngrößenverteilung. Die im Austrag zu erzielende Korngröße ist dabei von der Keimbilanz in der Wirbelschichtanlage abhängig. Diese wird wesentlich vom Gleichgewicht von Keimbildung durch Abrieb oder nicht treffende Sprühtropfen und dem Granulataufbau bestimmt. Gezielt kann die Korngröße durch die Wahl der Trocknungs- und der Sprühparameter sowie durch den Einsatz eines Zerhackers eingestellt werden. Die so erzeugten Granulate können durch eine klassierende Einrichtung (z.B. Sichter, Unterlaufwehr) in angestrebter Partikelgröße kontinuierlich aus dem Trocknungsraum ausgetragen werden. Die Partikelgröße des Produktes kann durch eine nachgeschaltete Siebung noch enger eingestellt werden. Das Überkorn der Siebung wird über eine Mühle zerkleinert und gemeinsam mit dem mittels Zyklon abgetrennten Feinkorn zurückgeführt (z.B. pneumatisch oder mittels Förderschnecke). Dies hat den Zweck, dass ausreichend Keime für die Granulation im Prozessraum vorhanden sind und kein Produkt verloren geht.

Die Sprühgranulation wird bei einer Temperatur in der Wirbelschicht von 60 bis 130°C, vorzugsweise von 80 bis 110° durchgeführt. Der Betriebsdruck hat auf den Prozess nur wenig Einfluss. Üblicherweise wird mit einem leichten Unterdruck von 5 bis 50 mbar, vorzugsweise von ca. 10 mbar gearbeitet, um den Austritt von Staub aus der Anlage zu vermeiden.

Die Sprühgranulation wird ferner bevorzugt über eine oder mehrere Düsen mit 1,0 bis 8,0 mm Düsendurchmesser, bei Düsendrucken von 1 bis 5,0 barü, idealerweise von 2,0 bis 3,0 barü durchgeführt. Die Sprühraten der eingesetzten Mischung sind in Abhängigkeit von der Anlagengröße zu wählen.

Als Düsentyp verwendet wird eine Dreistoffdüse (pneumatischer Zerstäuber), vorzugsweise mit kreisförmigem Vollkegel als Zerstäuberform (z.B. von Fa. Schlick).

Auf die in Schritt (a) oder (b) des erfindungsgemäßen Verfahrens erhaltenen Partikel kann ferner durch Besprühen mit einer wässrigen Lösung, enthaltend 30 bis 40 Gew.% Ammoniumsulfat unter gleichzeitigem Verdampfen des Wassers eine Ammoniumsulfat-Hüllschicht aufgebracht werden, wobei der Anteil der auf den Partikeln so gebildeten Ammoniumsulfat-Hüllschicht 5 bis 30 Gew.% bezogen auf das Gesamtgewicht der gebildeten Partikel beträgt. Der Vorteil dabei liegt vor allem darin, dass die Hüllschicht zusätzlich unangenehme Geruchskomponenten einschließt und auf diese Weise ein fast geruchsneutrales Produkt erzeugt wird. Das Besprühen der Partikel kann zweckmäßigerweise direkt in der zur Sprühgranulation verwendeten Vorrichtung mittels der vorhandenen Dreistoffdüse erfolgen. Es ist aber ebenfalls möglich, das Besprühen über eine Zweistoffdüse durchzuführen. Die zum Besprühen einzusetzende wässrige Ammoniumsulfatlösung kann identisch zur vorstehend erwähnten ersten wässrigen Lösung oder Suspension (i) sein, d.h. beispielsweise auch aus der Blausäureproduktion stammen.

Im kleineren Maßstab wird dabei die diskontinuierliche Fahrweise bevorzugt, d.h. die Beschichtung erfolgt nach dem Granulationsprozess. Im großtechnischen Produktionsmaßstab wird die kontinuierliche Fahrweise bevorzugt. Der Granulator wird dazu vorzugsweise in mehrere Zonen unterteilt. Die ersten Zonen werden für den Granulationsschritt (a) verwendet. Anschließend folgen eine Beschichtungs-Zone, eine Nachtrocknungszone und eine Kühlungszone zur entsprechenden Weiterverarbeitung des primär gebildeten Granulates. Die Nachtrocknung und die Kühlung können auch in separaten Anlagen (z.B. Fließbetttrockner, -kühler mit integrierten Heiz- bzw. Kühlregistern) erfolgen. Als Granulatoren eignen sich übliche Wirbelschicht- bzw. Fließbettanlagen.

Die nach dem erfindungsgemäßen Verfahren hergestellte, partikelförmige Zusammensetzung eignet sich zur Verwendung als Düngemittel oder Düngemittelzusatz, insbesondere als Stickstoff-/Kalium-/Schwefel (NKS)-Dünger zur Ausbringung auf Anbauflächen für Nutzpflanzen. Sie hat im Vergleich zur bisher als Flüssigdünger verwendeten Purge-Lösung eine deutlich höhere Wirkstoffkonzentration sowie bessere Lagereigenschaften und Produktstabilität. Das Produkt ist auch sicherheitstechnisch unbedenklich. Es ist z.B. kein Staub-Ex-Produkt. Die Notwendigkeit von Flüssigkeitstransport und -lagerung in entsprechenden Tanks oder Tankfahrzeugen entfällt außerdem. Das Produkt kann auch in kleineren Gebinden z.B. als Sackware oder in Big Bags an Kunden geliefert werden.

Die erfindungsgemäß hergestellte partikelförmige Zusammensetzung hat eine durchschnittliche Partikelgröße von ca. 1 bis 4 mm. Dies hat den Vorteil insbesondere gegenüber größeren Partikeln, dass sie auf den Anbauflächen relativ gleichmäßig verteilbar ist und somit eine Über- bzw. Unterdosierung bei der Anwendung als Feststoffdünger besser vermieden werden kann.

Die erfindungsgemäß durch Sprühgranulation mit Hilfe einer Dreistoffdüse hergestellte partikelförmige Zusammensetzung weist gegenüber einer mittels einer Zweistoffdüse hergestellten partikelförmige Zusammensetzung eine geringere Feuchteaufnahme bei Lagerung bei feuchtwarmen Bedingungen auf (Beispiel 2), sowie deutlich verringerte Verklumpungsneigung (Beispiel 3), was zu günstigeren Handhabungs- und Verarbeitungseigenschaften führt, wie z.B. zu verbesserten Fließeigenschaften.

Da bereits der Gehalt der erfindungsgemäßen Zusammensetzung an Ammoniumsulfat das Verklumpen der Partikel vermindert, ist die Beimengung eines Bindemittels bzw. Fließhilfsmittels in der Regel überflüssig. Sofern dennoch ein Bindemittel bzw. Fließhilfsmittel für die Herstellung der erfindungsgemäßen Düngemittelzusammensetzungen eingesetzt werden soll, wird dieses zweckmäßigerweise im Falle der diskontinuierlichen Fahrweise eines Wirbelschichtgranulators durch Mischen des Bindemittels bzw. Fließhilfsmittels mit dem Startmaterial in die Wirbelschicht eingebracht. Als Startmaterial (Trägermaterial) eignet sich insbesondere der Rückstand der eingedampften und getrockneten zweiten wässrigen Lösung oder Suspension (ii) bzw. der Mutterlauge (Purge-Lösung) aus dem eingangs beschriebenen Hydantoinhydrolysekreislauf, welcher vorher z.B. mit einer Siebmühle auf eine geeignete mittlere Korngröße von z.B. d50 = 150 µm zerkleinert wurde. Alternativ kann auch Methionin-Pulver (z.B. mit Korngröße d50 = 180 µm) oder Ekoperl - ein Ölbindemittel aus porösem vulkanischem Silikatgestein (Perlit) z.B. in der Körnung 0,125 bis 2 mm - als Startmaterial eingesetzt werden.

Als Bindemittel eignet sich z.B. poröses vulkanisches Silikatgestein (z.B. Perlit), gefällte oder pyrogene Kieselsäure oder poröses Karbonatgestein.

Im Falle der kontinuierlichen Fahrweise z.B. eines Produktionsverfahrens geschieht das Zumischen des Bindemittels bzw. Fließhilfsmittels zweckmäßigerweise mittels Injektor, Saugtaktförderung, pneumatischer Förderung, Zellenradschleuse, Rühr-Vorlagebehälter oder Förderschnecke.

**Beispiele**

**Beispiel 1**

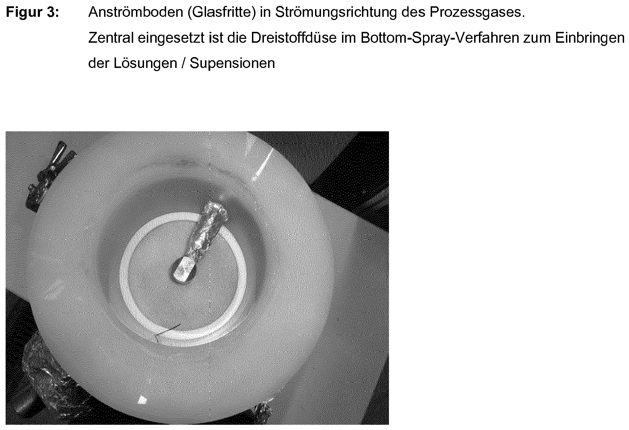

Die Sprühgranulationsversuche mit einer Dreistoffdüse wurden im Batch-Modus an einer Laborwirbelschichtgranulationsanlage (Glatt ProCell AGT150, siehe schematischer Aufbau in Figur 2) durchgeführt. Die Experimente bestätigen eine problemlose Herstellung von stabilen Granulaten ohne Anbackungen und Verkrustungen der Wirbelschichtkammer und der Dreistoffdüse (siehe Figur 3). Weiterhin zeigen die Versuche, dass die kurzen Mischzeiten ausreichend sind, um den Ammoniak zu binden und die Entstehung einer klebrigen Phase zu vermeiden.

**Geeignete Apparatur und allgemeine Beschreibung der Durchführung des Verfahrens**

Die verwendete Wirbelschichtgranulationsanlage der Firma Glatt (Typ ProCell) ist in Figur 1 schematisch dargestellt. Die zylindrische Prozesskammer ist 300 mm hoch und besitzt einen Durchmesser von 150 mm. Die Staubabscheidung der Prozessluft erfolgte mit innenliegenden Textilfiltern (6 Filtersäcke). Diese wurden im Gegenstrom zur Prozessluft durch einen Druckluftstoß abgereinigt. Alternativ zu einem Filterabscheider kann z.B. auch ein Zyklon eingesetzt werden. Das Prozessgas wurde aus dem zentralen Stickstoffnetz gespeist und durch einen Ventilator im Saugbetrieb durch die Anlage gefördert. Im Prozessraum herrschte daher Unterdruck (ca. -10 mbar). Vor Eintritt in die Prozesskammer wurde die Luft durch einen Elektroheizer (9 kW, Fa. Helios) direkt beheizt. Die erwärmte Luft durchströmte im Anschluss eine Glasfritte, die als Anströmboden der Partikel dient. Durch die enge Porenverteilung wird eine gleichmäßige Verteilung der Strömung erzielt. In dem aufwärts gerichteten Gasstrom wurden die Partikel im Prozessraum in einen fluidisierten Zustand versetzt und die versprühte Flüssigkeit durch den Wärmeeintrag der Trocknungsluft verdampft.

Der versprühte Feststoff bleibt dabei idealerweise auf den vorhandenen Partikeln haften, wodurch sich ein diskretes Partikelwachstum einstellen lässt. Die im Austrag zu erzielende Korngröße ist von der Keimbilanz in der Wirbelschichtanlage abhängig. Diese wird wesentlich vom Gleichgewicht von Keimbildung durch Abrieb oder nicht treffende Sprühtropfen und dem Granulataufbau bestimmt. Gezielt kann die Korngröße durch die Wahl der Trocknungs-, der Sprühparameter und durch den Einsatz eines Zerhackers in der Wirbelschicht eingestellt werden. Die so erzeugten Granulate können durch eine klassierende Einrichtung (z.B. Sichter, Überlaufwehr) in angestrebter Partikelgröße kontinuierlich aus dem Trocknungsraum ausgetragen werden.

Im Beispiel wurde die Granulation im Batch-Verfahren ohne einen kontinuierlichen Produktaustrag durchgeführt.

In die Glasfritte zentrisch eingelassen ist eine Dreistoffdüse, über die die wässrigen Lösungen / Suspensionen (Lösung oder Suspension (i) bzw. (ii), ggf. in aufkonzentrierter Form) zur Granulation der Partikel in die Wirbelschicht eingebracht werden können. Vereinfachend wird in der Beschreibung für den Begriff "Lösung oder Suspension (i)" bzw. "Lösung oder Suspension (ii)" auch der Begriff "Lösung i" bzw. "Lösung ii" gebraucht.

Das Einsprühen erfolgte mittels Bottom-Spray-Verfahren. Prozessgas und Sprühstrahl der Düse traten dabei im Gleichstrom von unten in die Prozesskammer ein. Die verwendete Dreistoffdüse ist ein Eigenbau der Evonik Industries AG, bestehend aus Originalteilen der Fa. Schlick (Drallkörper, Spindel und Luftkappe, Typ 946 S1, Fa. Düsen-Schlick GmbH). Die Dreistoffdüse weist ein verlängertes Mittelrohr auf, das Zentralrohr ist ca. 1 mm kürzer als im Auslieferungszustand der Fa. Düsen-Schlick. Die Luftkappe der Düse ist in Figur 2 nicht abgebildet. Figur 3 zeigt die Position der Düse im Anströmboden.

Für die Sprühgranulation in der vorstehend beschriebenen Apparatur gemäß Beispiel 1 wurden die wässrigen Lösungen in der nachfolgend genannten Zusammensetzung im Mischungsverhältnis Lösung (ii) : Lösung (i) von 1:2 eingesetzt.

| Wässrige Lösung (i): | 35 Gew.% Ammoniumsulfat |
| Wässrige Lösung (ii) | 4,6 Gew.% Methionin |
| | 6,3 Gew.% Methionylmethionin |
| | 9,7 Gew.% Kalium |

Lösung i und Lösung ii wurden jeweils aus einem Kunststoffkanister bei Raumtemperatur entnommen. Um die $NH_3$-Freisetzung zu vermeiden, wurde 20%ige Schwefelsäure in die erste wässrige Lösung oder Suspension (i) zugegeben. Diese Lösung i +$H_2SO_4$ wurde vor dem Versprühen in einem Mischbehälter und einem Magnetrührer homogenisiert. Die beiden Vorlagebehälter mit der Lösung i bzw. i + $H_2SO_4$ und Lösung ii wurden auf einer Waage (Messbereich bis 6 kg) platziert, um die Eindüsung (Sprührate) gravimetrisch über die Gewichtsabnahme des Vorlagebehälters zu bestimmen. Die Lösungen / Suspension wurden mit Hilfe der folgenden Pumpen aus den Vorlagen entnommen und in die Prozesskammer eingebracht:

| | Lösung ii (Purge-Lösung) | Lösung i (Ammoniumsulfatlsg.) + $H_2SO_4$ |
|---|---|---|
| Pumpe | Schlauchpumpe Watson & Marlow 503 U | Schlauchpumpe Watson & Marlow 505 U Kopf für konfektionierte Schläuche |
| Schlauch | Silikon, Øi 4,8*1,6 (Verder-Schlauch) | Øi 2,79 purple-white Marprene |

Die für den Prozess eingestellten Prozessparameter sind in der Tabelle 1 aufgelistet und wurden während des Versuches kontinuierlich mit den benannten Sonden/Sensoren erfasst.

Tabelle 1: Prozessparameter und deren Bestimmung.

| Prozess parameter | Anzeigewert | Sonde / Sensor |
|---|---|---|
| Gaseintrittstemperatur | °C | Thermoelement 2FK(J)AC10 (Eisen-Konstantan), Durchmesser 1 mm |
| Gasvolumenstrom | $m^3/h$ | Flügelrad-Strömungssensor FA (Zylindersonde), Typ ZS25GA-mn40/140/p6 170 mm, Fa. Höntzsch |
| Wirbelbetttemperatur | °C | Widerstandsthermometer PT 100, Klasse A, 4 Leiter |
| Massenstrom der eingedüsten Lösungen * | kg/h | Gewichtsabnahme der Vorlagebehälter (gravimetrisch); Lösung ii mittels Sartorius-Waage Universal U6100 / Messbereich bis 6 kg; Lösung i + $H_2SO_4$ mittels Sartorius Typ F 150 S-DJZ / Messbereich bis 150 kg; |
| = Separate Messung, keine Integration ins Prozessleitsystem der Wirbelschichtanlage | | |

**Vorbereitung der Materialien**

Die zweite wässrige Lösung oder Suspension (ii) wurde ohne eine vorherige Aufbereitung direkt versprüht. Eine Voreindampfung von ii ist immer möglich und aus energetischen Gründen im großtechnischen Verfahren zu empfehlen.
Bereitstellung der Lösung i + $H_2SO_4$ durch Zugabe von Schwefelsäure in die erste wässrige Lösung oder Suspension (i)

1. Lösung i wurde vorgelegt,

2. Zugabe von 20%iger Schwefelsäure bis zu einer Konzentration von 4.4 Gew.% $H_2SO_4$ in der finalen Mischung (entspricht Lösung i + $H_2SO_4$)

Beide Lösungen wurden separat voneinander mittels Dreistoffdüse in den Prozessraum und das vorhandene Startmaterial versprüht. Als Startmaterial diente eine Mischung aus der getrockneten Lösung i und ii. Das getrocknete Startmaterial wurde vor Verwendung mit Hilfe eines Siebgranulators FGS der Fa. Erweka, Heusenstamm auf eine Korngröße von 600 $\mu$m zerkleinert. Anschließend wurden 500g eingewogen und als Startfüllung eingesetzt. Alternativ kann auch Methionin-Pulver (Korngröße d50 = 180 $\mu$m) oder Ekoperl - ein Ölbindemittel aus porösem vulkanischem Silikatgestein (Perlit) in der Körnung von z.B. 0,125 bis 2 mm - als Startmaterial eingesetzt werden.

**Vorbereitung des Apparates**

Das Startmaterial im Wirbelschichtgranulator wurde bei einer Gaseingangstemperatur von 120°C auf eine Produkttemperatur von 80-90°C aufgeheizt. Anschließend wurden die Lösungen ii und i + $H_2SO_4$ mittels Schlauchpumpen zur Düse gefördert und von dort in die Wirbelschicht auf das Startmaterial versprüht.

Die Fluidisierung erfolgte mit einem Luftvolumenstrom zwischen 40 - 200 m$^3$/h. Dies entspricht einer Anströmgeschwindigkeit von 0,6 - 3,1 m/s bei der gegebenen Anströmfläche (0,018 m$^2$). Der Gasvolumenstrom wurde am Eintritt der Anlage mittels Flügelrad gemessen (siehe Figur 1). Die eintretende Luft war hier zunächst kalt und trocken. Sie passierte im Anschluss einen Elektroheizer. Unterhalb des Anströmbodens der Anlage befand sich ein Temperaturfühler, der die Gastemperatur unmittelbar unter dem Anströmboden und damit vor Eintritt des Gases in die Prozesskammer bestimmt (Figur 1, Messstelle Temperatur 1). Diese Gastemperatur dient als Regelgröße und wurde vorgegeben. Im Abgleich mit dem Messwert wurde damit der Energieeintrag über den Gaserhitzer gesteuert und die Prozessluft auf die am Anströmboden vorgegebene Temperatur erwärmt.

Je nach eingebrachter Heizenergie verändert sich die Temperatur der Luft am Anströmboden. In Folge dessen wurde der Gasvolumenstrom durch die Anlage entsprechend angepasst, so dass unter Berücksichtigung der Gasdichte am Anströmboden ein konstanter Volumenstrom anlag. Dies geschah automatisiert durch das Prozessleitsystem der Wirbelschichtanlage, das den saugseitig angebrachten Ventilator steuerte.

Beispiel 1 wurde in der oben beschriebenen Apparatur gemäß Figur 1 in diskontinuierlicher Fahrweise durchgeführt. Dabei wurden die folgenden Versuchsparameter eingestellt:

**Eingestellte Parameter bei der Wirbelschicht-Sprühgranulation:**

$$T \text{ Zuluft} = 100 - 200°C$$

$$T \text{ Wirbelbett} = 60 - 110°C$$

$$T \text{ Abluft} = 60 - 130°C$$

$$\text{Sprühraten} = 1 \text{ kg/h der Lösung ii und } 2.6 \text{ kg/h der Lösung i} + H_2SO_4$$

Düsendruck (Dreistoffdüse) = 1,2 bar
Volumenstrom Trocknungsluft = 40 - 200 m$^3$/h. Dies entspricht einer Anströmgeschwindigkeit von 0,6 - 3,1 m/s bei der gegebenen Anströmfläche (0,018 m$^2$)
Anlagendruck oberhalb des Siebbodens = 10 mbar unterhalb Atmosphärendruck
Mittlere Verweilzeit: 0,5 - 3 h.

**Auswertung und Ergebnisse: Produkteigenschaften:**

**Tabelle 2:** Produkteigenschaften des erhaltenen Sprühgranulates

| Nr. | SG [kg/l] | Durch-messer (mm) | Staub-neigung | Riesel-fähigkeit | Verklump-ungsneig-ung | Zusammen-setzung (K : N : S) | Geruch | pH |
|---|---|---|---|---|---|---|---|---|
| 1 | 0,75 | 1-2 | gering | gut | gering | 11 : 14 : 19 | gering | 4,1 |

Der pH-Wert der partikelförmigen Zusammensetzung wurde gemessen an einer 10%igen Lösung des erhaltenen Sprühgranulates mit Wasser bei Raumtemperatur mit Hilfe einer Glas-pH-Elektrode mit Flüssig-Elektrolyt-Füllung in Form einer 3-molaren KCl-Lösung.

**Beispiel 2: Hygroskopizitätstest an den Sprühgranulaten**

Um die Wasseraufnahme der zu prüfenden Substanz zu bestimmen, wurde diese über einen definierten Zeitraum einem definierten Standardklima von 40 °C und 75 % rel. Luftfeuchte ausgesetzt. Die Wasseraufnahme wurde anschließend gravimetrisch bestimmt.

Dazu verwendet wurden eine Klimakammer (z.B. VÖTSCH, VC0033), flache Wägegläschen mit Glasdeckel (Durchmesser ca. 5 cm) und eine Analysenwaage (Ablesbarkeit 0,0001 g).

Dabei wurden jeweils 5 g der zu bestimmenden, homogenen Substanz genau eingewogen und in einem offenen Wägegläschen unter den definierten Standardklimabedingungen in der Klimakammer für 1 h bzw. 4 h gelagert. Die nach Entnahme aus der Klimakammer wiederverschlossenen Wägegläschen wurden jeweils ausgewogen und die Gewichtszunahme (= Feuchteaufnahme) bestimmt.

Das erfindungsgemäß mit Hilfe einer Dreistoffdüse nach Beispiel 1 hergestellte Sprühgranulationsprodukt wurde dabei verglichen mit einem Sprühgranulationsprodukt, das zuvor mit Hilfe einer Zweistoffdüse nach einem in EP 16176371.9 beschrieben Verfahren (Beispiel 5) hergestellt worden war.

Das mittels Zweistoffdüse hergestellte Sprühgranulat zeigte eine Feuchteaufnahme von 2 Gew.% nach 1h und 5,3 Gew.% nach 4 h.

Das mittels Dreistoffdüse hergestellte Sprühgranulat zeigte eine Feuchteaufnahme von 2 Gew.% nach 1h und 4,9 Gew.% nach 4 h.

Die Hygroskopizität (Wasseraufnahme) ist bei mittels Dreistoffdüse hergestelltem Sprühgranulat kleiner und damit günstiger als bei mittels Zweistoffdüse hergestelltem Sprühgranulat.

Ganz analog wurden vergleichende Lagertests mit dem Produkt aus Beispiel 1 und dem Produkt aus Beispiel 5 in EP 16176371.9 in einem definierten Standardklima von 25 °C und 60 % rel. Luftfeuchte über einen Zeitraum von 166 Stunden durchgeführt und die Wasseraufnahme von Zeit zu Zeit gravimetrisch bestimmt. Die Ergebnisse sind in Figur 5 abgebildet und zeigen ebenfalls die geringere Wasseraufnahme bei mittels Dreistoffdüse hergestelltem Sprühgranulat im Vergleich zu mittels Zweistoffdüse hergestelltem Sprühgranulat.

**Beispiel 3: Verklumpungsneigung via Kratztest**

Bei der"Kratztest"-Methode mit dem Texture-Analyser wird mit einem Edelstahlrotor in eine Probe rotierend eingetaucht und diese abgerieben / "abgekratzt". Die entstehenden Kräfte wirken auf das empfindliche Wägesystem in der Bodenplatte. Je starker die Verbackung , desto höher die gemessene Kraft und somit die Verklumpungsneigung des Produkts.

Dazu verwendet wurden eine Trockenschrank (z.B. MEMMERT, Typ UM 600), eine Klimakammer (z.B. VOTSCH , Typ VC 0033), Metallzylinder mit passender Metallhülse (Zylinder: 0 = 50 mm, h = 80 mm, m = 1,3 kg, VA-Stahl), eine Teflonscheibe als Stempelunterlage (80 x 80 mm, Stärke 5 mm), eine Stempel-Beschwerung als Auflagegewicht (Metallzylinder, m = 4 kg)

TA.XTPlus Texture Analyser (Fa. STABLE MICRO SYSTEMS, Vertrieb durch Fa. Winopal GmbH, Ahnsbeck) mit Software und Zubehör, ein Edelstahlrotor (Durchmesser = 40 mm), Haltevorrichtung für die Metallhülse und Teflonscheibe , ein PC zur Datenerfassung.

Die **Durchführung** der Messungen geschah nach folgendem Ablaufschema:

Temperierung von Hülse, Zylinder, Gewicht und Teflonscheibe bei 40 °C im Trockenschrank

- Hülse wird auf die Teflonscheibe gestellt

- 30 g homogenisierte Substanz wird eingefüllt (parallele 5-fach- Bestimmung wird durchgeführt) und die Oberfläche mit einem Spatel jeweils geebnet
- der Zylinder wird in die Hülse eingesetzt und alles in die Klimakammer gestellt
- Eine 4 kg Stempel-Beschwerung wird auf den Zylinder aufgesetzt
- Die beschwerten Zylinder werden nun für 24 Stunden bei 40 °C / 75 % rel. Feuchte in der Klimakammer gelagert und auf diese Weise bewittert.
- Anschließend wird die Stempel-Beschwerung abgenommen
- Hülse und Zylinder werden zusammen mit der Teflonscheibe herausgenommen und ca. 2 Stunden auf Raumtemperatur abgekühlt
- Der Zylinder wird aus der Hülse gezogen.

- Die Hülse verbleibt auf der Teflonplatte, wird auf die Haltevorrichtung des Texture-Analysers überführt und mit dem Klettband gesichert
- Mit der Software wird der Messvorgang wie folgt automatisch gestartet (Parameter Eindringgeschwindigkeit 2 mm/s; Rotorgeschwindigkeit 10 U/min): Beim Absenken des Rotors wird die Tablettenhöhe automatisch gemessen und die Eindringtiefe mit 75 % der Tablettenhöhe festgelegt. Die durch das "Kratzen" entstehenden Kräfte werden aufgezeichnet und für die Auswertung der Kraftbereich von 20 bis 60 % (des Weges) verwendet.

**Auswertung:** Zur Bestimmung der Verklumpungsneigung diente der Mittelwert des Kraftbereichs jeder Messung, vereinfacht in [Newton/100] angegeben. Die Ergebnisse der Mehrfachbestimmung wurden wiederrum gemittelt. Umso geringer diese Werte ausfallen, desto geringer ist die Verklumpungsneigung.

Das erfindungsgemäß mit Hilfe einer Dreistoffdüse nach Beispiel 1 hergestellte Sprühgranulationsprodukt wurde dabei wiederum verglichen mit einem Sprühgranulationsprodukt, das zuvor mit Hilfe einer Zweistoffdüse nach einem in EP 16176371.9, Beispiel 5 beschriebenen Verfahren hergestellt worden war.

Das mittels Zweistoffdüse hergestellte Sprühgranulat zeigte eine Verklumpungsneigung von 228 Newton/100. Das mittels Dreistoffdüse hergestellte Sprühgranulat zeigte eine Verklumpungsneigung von 155 Newton/100.

Die Verklumpungsneigung ist beim mittels Dreistoffdüse hergestellten Sprühgranulat deutlich kleiner und damit entsprechend günstiger als beim mittels Zweistoffdüse hergestellten Sprühgranulat. Dieses Produkt hat somit eine höhere Toleranz gegenüber feuchtheißem Klimata als herkömmliches Sprühgranulat und ist folglich leichter handhabbar.

**Beschreibungen der Figuren**

### Beschreibung zu Figur 1

| | |
|---|---|
| 1 | Staubabscheider (z.B. Zyklon, Filter, Nasswäscher) |
| 2 | Pumpe (z.B. Zwangsförderpumpe) |
| 3 | Düsenluft (z.B. Luft, Stickstoff) |
| 4 | Heizung (z.B. elektrisch, Dampf, Gasfeuerung) |
| 5 | Zuluftgebläse |
| 6 | Siebboden, Conidurblech |
| 7 | Wirbelschicht, Fließbett |
| 8 | Abluftgebläse |
| 9 | erste wässrige Lösung oder Suspension (i) |
| 10 | zweite wässrige Lösung oder Suspension (ii) |
| 11 | Messstelle Gasvolumenstrom, Flügelrad Anemometer |
| 12 | Messstelle Temperatur 1 (Gaseintrittstemperatur) |
| 13 | Messstelle Temperatur 2 (Wirbelschichttemperatur) |
| 14 | Messstelle Temperatur 3 (Ablufttemperatur) |

### Beschreibung zu Figur 2

| | |
|---|---|
| 1 | Düsenluft (z.B. Luft, Stickstoff) |
| 2 | erste wässrige Lösung oder Suspension (i) |
| 3 | zweite wässrige Lösung oder Suspension (ii) |
| 4 | Luftkappe |
| 5 | Sprühnebel |

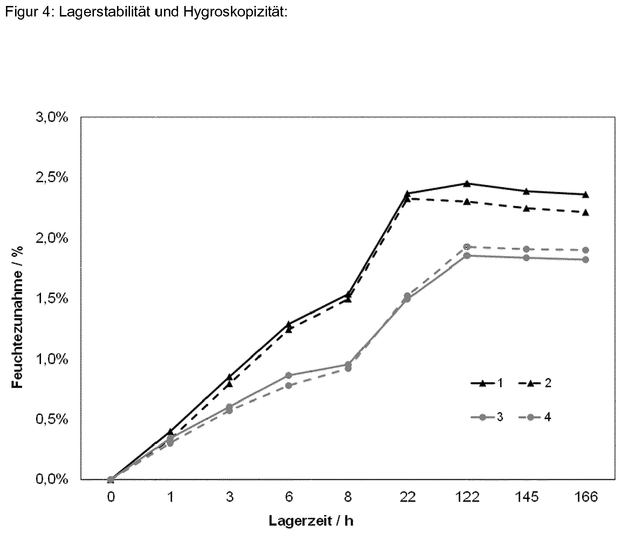

### Beschreibung zu Figur 4

| | |
|---|---|
| 1: | Vergleichsprodukt aus Beispiel 5 der EP-Anmeldung mit Anmeldenummer 16176371.9 (1. Messung) |
| 2: | Vergleichsprodukt aus Beispiel 5 der EP-Anmeldung mit Anmeldenummer 16176371.9 (2. Messung) |
| 3: | Produkt hergestellt mittels 3-Stoffdüse (1. Messung) Beispiel 1 |
| 4: | Produkt hergestellt mittels 3-Stoffdüse (2. Messung) Beispiel 1 |

**EP 3 461 803 A1**

**Patentansprüche**

1. Verfahren zur Herstellung einer Methionin, Methionylmethionin, Kaliumsalz und Ammoniumsulfat enthaltenden, partikelförmigen Zusammensetzung durch Sprühgranulation, **dadurch gekennzeichnet dass**

   (a) eine erste wässrige Lösung oder Suspension (i), enthaltend

   30 bis 40 Gew.% Ammoniumsulfat,
   eine zweite wässrige Lösung oder Suspension (ii), enthaltend 2 bis 6 Gew.% Methionin,
   4 bis 8 Gew.% Methionylmethionin und
   6 bis 14 Gew.% Kalium in Form von Kaliumsalz und
   ein Gas
   über eine Dreistoffdüse in eine Wirbelschicht versprüht werden unter gleichzeitigem Verdampfen von Wasser in der Wirbelschicht und dabei Rohpartikel erzeugt werden, und optional

   (b) die in Schritt (a) erhaltenen Rohpartikel getrocknet werden.

2. Verfahren nach Anspruch 1, bei welchem eine zweite wässrige Lösung oder Suspension (ii),
   mit einem Gehalt von 3 bis 5 Gew. % Methionin und/oder
   5 bis 7 Gew.% Methionylmethionin und/oder
   8 bis 12 Gew.% Kalium in Form von Kaliumsalz eingesetzt wird.

3. Verfahren nach Anspruch 1 oder 2, bei welchem die zweite wässrige Lösung oder Suspension (ii) in einem Gewichtsverhältnis zur ersten wässrigen Lösung oder Suspension (i) von
   1,0/0,5 bis 1,0/3,0 eingesetzt wird.

4. Verfahren nach einem der Ansprüche 1 bis 3, **dadurch gekennzeichnet dass** Luft, Stickstoff oder $CO_2$ als Gas verwendet werden.

5. Verfahren nach einem der Ansprüche 1 bis 4, **dadurch gekennzeichnet dass** der Schritt (a) des Verfahrens in einer Wirbelschicht bei einer Temperatur in der Wirbelschicht von 60 bis 130°C durchgeführt wird.

6. Verfahren nach einem der Ansprüche 1 bis 5, ferner umfassend den Schritt der Herstellung der Ammoniumsulfat enthaltenden, ersten wässrigen Lösung oder Suspension (i) im Wesentlichen durch Behandeln eines bei der Herstellung von Blausäure aus Methan und Ammoniak anfallenden, Blausäure und Ammoniak enthaltenden Gasgemisches mit wässriger Schwefelsäure und anschließender Neutralisation der erhaltenen wässrigen Lösung mit Ammoniak.

7. Verfahren nach einem der Ansprüche 1 bis 6, ferner umfassend den Schritt der Herstellung der zweiten wässrigen Lösung oder Suspension (ii) durch Abtrennung einer Mutterlauge, welche bei einem Verfahren zur Herstellung von Methionin entsteht, welches mindestens die Schritte
   Hydrolyse von 5-(2-Methylmercaptoethyl)-hydantoin mit Kaliumcarbonat, Kaliumhydrogencarbonat zu wässrigem Kaliummethioninat,
   Neutralisation von wässrigem Kaliummethioninat mit Kohlendioxid unter Bildung von Methionin und
   anschließende Kristallisation des Methionins umfasst.

8. Verfahren nach einem der Ansprüche 1 bis 7, bei welchem die erste wässrige Lösung oder Suspension (i) zusätzlich mit so viel Schwefelsäure versetzt wird, dass nach der Sprühgranulation eine partikelförmigen Zusammensetzung erhalten wird, die nach Auflösen in Wasser einen pH-Wert von 3 bis 6, vorzugsweise von 3,5 bis 5,5, aufweist, gemessen an einer 10 Gew.%igen wässrigen Lösung der partikelförmigen Zusammensetzung bei Raumtemperatur mit einer Glas-pH-Elektrode mit Flüssig-Elektrolyt-Füllung in Form einer 3-molaren KCl-Lösung.

9. Verfahren nach einem der Ansprüche 1 bis 8, bei welchem ferner die in Schritt (a) eingesetzte wässrige Lösung oder Suspension (ii) vor Schritt (a) durch Verdampfen von Wasser zu einer wässrigen Suspension mit bis zu 70 Gew.% Feststoffgehalt aufkonzentriert wird.

10. Verfahren nach einem der Ansprüche 1 bis 9, bei welchem auf die in Schritt (b) erhaltenen Partikel durch Besprühen mit einer wässrigen Lösung enthaltend 30 bis 40 Gew.% Ammoniumsulfat unter gleichzeitigem Verdampfen des

11

Wassers eine Ammoniumsulfat-Hüllschicht aufgebracht wird, wobei der Anteil der auf den Partikeln gebildeten Ammoniumsulfat-Hüllschicht 5 bis 30 Gew.% bezogen auf das Gesamtgewicht der gebildeten Partikel beträgt.

11. Verfahren nach einem der Ansprüche 1 bis 10, wobei das Kaliumsalz als mindestens ein Salz einer anorganischen oder organischen Säure vorliegt.

12. Verfahren nach Anspruch 11, **dadurch gekennzeichnet, dass** es sich beim Kaliumsalz um mindestens ein Kaliumsalz ausgewählt aus der Gruppe umfassend Ameisensäure, Essigsäure, Propansäure, 2-Hydroxypropansäure, 2-Hydroxy-4-methylthiobutan-säure, Methionin, Methionylmethionin, $KHCO_3$, $K_2CO_3$, $KHSO_4$ und $K_2SO_4$ handelt.

13. Verwendung einer Zusammensetzung hergestellt nach einem Verfahren gemäß einem der Ansprüche 1 bis 12 als Düngemittel oder Düngemittelzusatz.

**Figur 1:** Apparatur zur Durchführung einer Sprühgranulation

**Figur 2**: Verwendete Dreistoffdüse

**Figur 3:** Anströmboden (Glasfritte) in Strömungsrichtung des Prozessgases. Zentral eingesetzt ist die Dreistoffdüse im Bottom-Spray-Verfahren zum Einbringen der Lösungen / Supensionen

Figur 4: Lagerstabilität und Hygroskopizität:

**Europäisches Patentamt**
**European Patent Office**
**Office européen des brevets**

# EUROPÄISCHER RECHERCHENBERICHT

Nummer der Anmeldung

EP 17 19 4295

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| E | WO 2018/001988 A1 (EVONIK DEGUSSA GMBH [DE]) 4. Januar 2018 (2018-01-04) * das ganze Dokument * ----- | 1-13 | INV. C05C3/00 C05F11/10 |
| A | EP 1 233 071 A2 (AJINOMOTO KK [JP]) 21. August 2002 (2002-08-21) * Seite 10 * ----- | 1-13 | |

RECHERCHIERTE SACHGEBIETE (IPC)

C05C
C05F

Der vorliegende Recherchenbericht wurde für alle Patentansprüche erstellt

| Recherchenort | Abschlußdatum der Recherche | Prüfer |
|---|---|---|
| Den Haag | 5. März 2018 | Cardin, Aurélie |

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 17 19 4295

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

05-03-2018

| Im Recherchenbericht angeführtes Patentdokument | Datum der Veröffentlichung | Mitglied(er) der Patentfamilie | Datum der Veröffentlichung |
|---|---|---|---|
| WO 2018001988 A1 | 04-01-2018 | KEINE | |
| EP 1233071 A2 | 21-08-2002 | AT 315096 T | 15-02-2006 |
| | | AU 782737 B2 | 25-08-2005 |
| | | BR 0200489 A | 01-10-2002 |
| | | CN 1377971 A | 06-11-2002 |
| | | DE 60208453 T2 | 28-09-2006 |
| | | EP 1233071 A2 | 21-08-2002 |
| | | ES 2253458 T3 | 01-06-2006 |
| | | JP 4292724 B2 | 08-07-2009 |
| | | JP 2002249390 A | 06-09-2002 |
| | | KR 20020068292 A | 27-08-2002 |
| | | RU 2291139 C2 | 10-01-2007 |
| | | US 2003172698 A1 | 18-09-2003 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5770769 A
- WO 2013030068 A2
- EP 1564208 A1
- EP 716640 B1
- EP 16176371 A
- US 8802020 B2

**In der Beschreibung aufgeführte Nicht-Patentliteratur**

- **C.C. MITCHEL ; A.E. HILTBOLD.** *Journal of Plant Nutrition,* 1994, vol. 17 (12), 2119-2134
- **F. ENDTER.** *Chemie-Ing.-Techn.,* 1958, vol. 30 (5), 305-310